# EUROPEAN PATENT SPECIFICATION

(11) **EP 1 011 489 B1**
(45) Date of publication and mention of the grant of the patent: **12.10.2011**
(21) Application number: 98907627.8
(22) Date of filing: 24.02.1998
(51) Int. Cl.: A61B 18/02

(54) **APPARATUS FOR LINEAR ABLATION**
GERÄT ZUR LINEAREN ABLATION
APPAREIL POUR ABLATION LINEAIRE

(30) Priority: 27.02.1997 US 807382; 11.07.1997 US 893825
(43) Date of publication of application: 28.06.2000
(60) Divisional application: 10179729.8
(73) Proprietor: MEDTRONIC CRYOCATH LP, Toronto, ON M5L 1B9 (CA)
(72) Inventor: ARLESS, Steven, G., Beaconsfield, Québec H92 4N4 (CA); MILDER, Fredric, L., Brookline, MA 02146 (US); SPECTOR, Kenneth, A., Framingham, MA 01701 (US); WITTENBERGER, Dan, Montreal, Québec H3X 2R5 (CA); SANTOIANNI, Domenic, N., St. Leonard, Québec H1R 1R6 (CA); LUECKGE, Claudia, Montreal, Québec H1T 1C4 (CA)
(74) Representative: Modiano, Micaela Nadia
(86) International application number: PCT/US1998/003674
(87) International publication number: WO 1998/037822

(56) References cited:
- US-A- 3 910 277
- US-A- 4 709 698
- US-A- 4 998 916
- US-A- 5 281 213
- US-A- 5 324 286
- US-A- 5 452 582
- US-A- 5 517 898
- US-A- 5 624 392

## Description

### FIELD OF THE INVENTION

The invention relates to catheters, and more particularly to cryosurgical catheters used for tissue ablation.

### BACKGROUND OF THE INVENTION

Many medical procedures are performed using minimally invasive surgical techniques, wherein one or more slender implements are inserted through one or more small incisions into a patient's body. With respect to ablation, the surgical implement can include a rigid or flexible structure having an ablation device at or near its distal end that is placed adjacent to the tissue to be ablated. Radio frequency energy, microwave energy, laser energy, extreme heat, and extreme cold can be provided by the ablation device to kill the tissue.

With respect to cardiac procedures, a cardiac arrhythmia can be treated through selective ablation of cardiac tissue to eliminate the source of the arrhythmia. A popular minimally invasive procedure, radio frequency (RF) catheter ablation, includes a preliminary step of conventional electrocardiographic mapping followed by the creation of one or more ablated regions (lesions) in the cardiac tissue using RF energy. Multiple lesions are frequently required because the effectiveness of each of the proposed lesion sites cannot be predetermined due to limitations of conventional electrocardiographic mapping. Often, five lesions, and sometimes as many as twenty lesions may be required before a successful result is attained. Usually only one of the lesions is actually effective; the other lesions result in unnecessarily destroyed cardiac tissue.

Deficiencies of radio frequency ablation devices and techniques have been overcome by using cold to do zero degree or ice mapping prior to creating lesions, as taught in U.S. Patent Nos. 5,423,807; and 5,281,213; and 5,281,215. However, even though combined cryogenic mapping and ablation devices permit greater certainty and less tissue damage than RP devices and techniques, both the cryogenic and the RF devices are configured for spot or roughly circular tissue ablation.

Spot tissue ablation is acceptable for certain procedures. However, other procedures can be more therapeutically effective if multiple spot lesions along a predetermined line, or a single elongate or linear lesion is created in a single ablative step. Radio frequency ablation devices are known to be able to create linear lesions by dragging the ablation tip along a line while it is active. However, no cryogenic devices are known that are optimizes for, or which are even minimally capable of, creating an elongate lesion. US 5,342,301 discloses lumens of the multi-lumen balloons that include: means for additional dilatation of a body cavity by inflating and deflating secondary, tertiary or other balloon lumens; providing working channels to contain sensors, such as thermocouple or fiber optics, or to house guide wires; channels for the delivery of fluids, medicine or drugs to the area under the balloon or to regions beyond the balloon; working channels to house laser or optical fibers or a heating wire used to cauterize tissue; channels used for biopsy or other sampling procedures; channels used for drainage, for example as a urology drain; and channels used to circulate heat transfer fluids for cooling or heating purposes, such as freezing of select areas with liquid nitrogen or heating select areas with heated fluid such as water or saline.

### SUMMARY OP THE INVENTION

The present invention provides a cryogenic ablation system including a cryosurgical catheter that is particularly well suited for creating elongate lesions that hoine the features set forth in claim 1. In an Exemplary embodiment, a cryogenic catheter includes a flexible member having an elongate, thermally-transmissive region and a cryogenic fluid path through the flexible member to the thermally-transmissive region. The thermally-transmissive region can be deformable

Further, preferred embodiments are disclosed in the dependent claims.

The thermally-transmissive region according to the invention is defined by a helical coil that is at least partially embedded in the flexible member. The helical coil can also define at least a portion of the cryogenic fluid path through the flexible member and can include a gas expansion or boiling chamber.

The cryogenic catheter of the invention can be a component in a cryogenic system that further includes a cryogenic fluid supply in communication with the cryogenic catheter, and a fluid controller interposed between the cryogenic catheter and the cryogenic fluid supply for regulating the flow of the cryogenic fluid into the cryogenic catheter. The cryogenic fluid can be a gas or a liquid.

### BRIEF DESCRIPTION OF THE DRAWINGS

A more complete understanding of the present invention and the attendant advantages and features thereof will be more readily understood by reference to the following detailed description when considered in conjunction with the accompanying drawings wherein:
FIG. 1 is a schematic illustration of an embodiment of a cryosurgical system in accordance with the invention;
FIG. 2 is a sectional view of a heart muscle showing placement of the catheter of FIG. 1;
FIG. 3 illustrates a tip region of a catheter;
FIG. 4 illustrates an alternative embodiment of the catheter of FIG. 3;
FIG. 5 illustrates yet another embodiment of the catheter;
FIG. 6 illustrates a deformable tip for a catheter;
FIG. 7 illustrates an embodiment of the catheter according to the invention invention
FIG. 8 is a sectional view of the catheter of FIG. 7 taken along line 8-8;
FIG. 9 is a sectional view of an alternative embodiment of the linear ablation catheter illustrated in FIG. 7;
FIG. 10 illustrates an expansion chamber within a portion of a helical coil;
FIG. 11 illustrates a portion of a catheter having an elongate, thermally-transmissive strip;
FIG. 12 is a sectional view of the catheter of FIG. 3 taken along line 12-12;
FIG. 13 is a sectional view of the catheter of FIG. 3 taken along line 13-13;
FIGS. 14-16 are sectional views of additional catheter embodiments;
PIG. 17 illustrates an inner face of a flexible catheter member;
FIG. 18 depicts yet another form of a catheter;
FIG. 19 is a table illustrating cooling performance of a catheter in accordance with the invention;
FIG. 20 is a sectional view of another catheter embodiment;
FIG. 21 is a sectional view of a portion of the catheter of FIG. 20;
FIG. 22 is a detailed view of an area of the catheter portion illustrated in FIG. 21;
FIG. 23 is an illustration of yet another catheter embodiment; and
FIG. 24 depicts still another catheter embodiment.

### DETAILED DESCRIPTION OF THE INVENTION

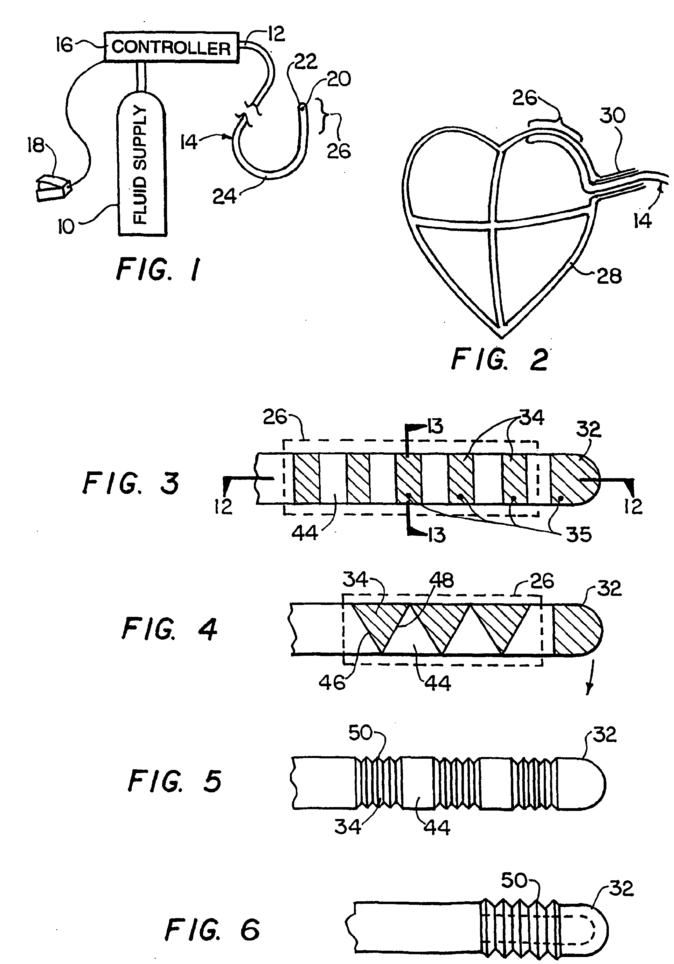

The embodiments of figures 3-6, 11-18 and 20-24 are not covered by the claims and are presented not as embodiments of the claimed invention, They are only disclosed as examples which are useful to understand the invention.

FIG. 1 is a schematic illustration of a cryosurgical system in accordance with the invention. The system includes a supply of cryogenic or cooling fluid 10 in communication with the proximal end 12 of a flexible catheter 14. A fluid controller 16 is interposed or in-line between the cryogenic fluid supply 10 and the catheter 14 for regulating the flow of cryogenic fluid into the catheter in response to a controller command. Controller commands can include programmed instructions, sensor signals, and manual user input. For example, the fluid controller 16 can be programmed or configured to increase and decrease the pressure of the fluid by predetermined pressure increments over predetermined time intervals. In another exemplary embodiment, the fluid controller 16 can be responsive to input from a foot pedal 18 to permit flow of the cryogenic fluid into the catheter 14. One or more temperature sensors 20 in electrical communication with the controller 16 can be provided to regulate or terminate the flow of cryogenic fluid into the catheter 14 when a predetermined temperature at a selected point or points on or within the catheter is/are obtained. For example a temperature sensor can be placed at a point proximate the distal end 22 of the catheter and other temperature sensors 20 can be placed at spaced intervals between the distal end of the catheter and another point that is between the distal end and the proximal end.

The cryogenic fluid can be in a liquid or a gas state. An extremely low temperature can be achieved within the catheter, and more particularly on the surface of the catheter by cooling the fluid to a predetermined temperature prior to its introduction into the catheter, by allowing a liquid state cryogenic fluid to boil or vaporize, or by allowing a gas state cryogenic fluid to expand. Exemplary liquids include chlorodifluoromethane, polydimethylsiloxane, ethyl alcohol, HFC's such as AZ-20 (a 50-50 mixture of difluoromethane & pentafluoroethane sold by Allied Signal), and CFC's such as DuPont's Freon. Exemplary gasses include nitrous oxide, and carbon dioxide.

The catheter 14 includes a flexible member 24 having a thermally-transmissive region 26 and a fluid path through the flexible member to the thermally-transmissive region. A fluid path is also provided from the thermally-transmissive region to a point external to the catheter, such as the proximal end 12. Although described in greater detail below, exemplary fluid paths can be one or more channels defined by the flexible member 24, and/or by one or more additional flexible members that are internal to the first flexible member 24. Also, even though many materials and structures can be thermally conductive or thermally transmissive if chilled to a very low temperature and/or cold soaked, as used herein, a "thermally-transmissive region" is intended to broadly encompass any structure or region of the catheter 14 that readily conducts heat.

For example, a metal structure exposed (directly or indirectly) to the cryogenic fluid path is considered a thermally-transmissive region 26 even if an adjacent polymeric or latex catheter portion also permits heat transfer, but to a much lesser extent than the metal. Thus, the thermally-transmissive region 26 can be viewed as a relative term to compare the heat transfer characteristics of different catheter regions or structures. A thermally-transmissive region or element is not intended to encompass a structure that is excited by RF or other energy source to a point where it begins to radiate heat, for example.

Furthermore, while the thermally-transmissive region 26 can include a single, continuous, and uninterrupted surface or structure, it can also include multiple, discrete, thermally-transmissive structures that collectively define a thermally-transmissive region that is elongate or linear. Depending on the ability of the cryogenic system, or portions thereof, to handle given thermal loads, the ablation of an elongate tissue path can be performed in a single or multiple cycle process without having to relocate the catheter one or more times or drag it across tissue. Additional details of the thermally-transmissive region 26 and the thermal transfer process are described in greater detail below.

In exemplary embodiments of the invention, the thermally-transmissive region 26 of the catheter 14 is deformable. An exemplary deformation is from a linear configuration to an arcuate configuration and is accomplished using mechanical and/or electrical devices known to those skilled in the art. For example, a wall portion of the flexible member 24 can include a metal braid to make the catheter torqueable for overall catheter steering and placement. Additionally, a cord, wire or cable can be incorporated with, or inserted into, the catheter for deformation of the thermally transmissive region 26.

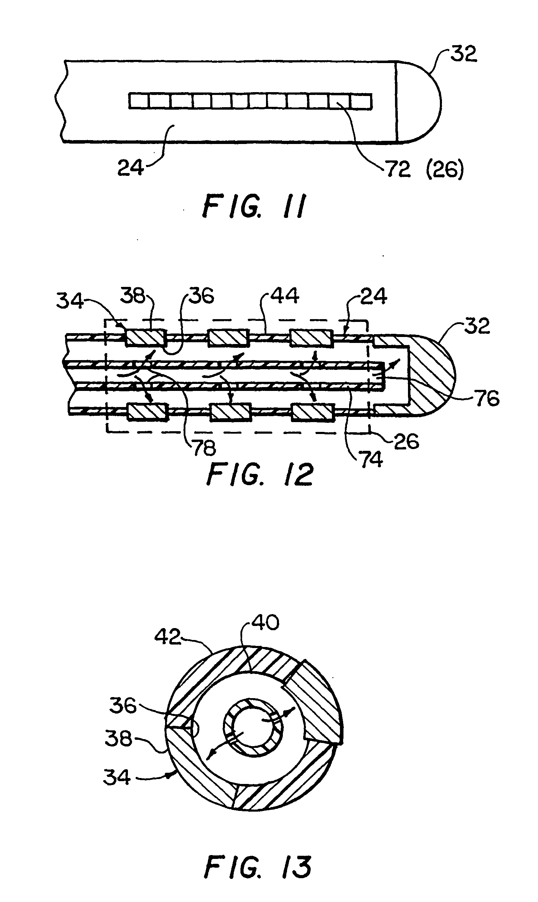

The cryogenic system of FIG. 1 is better understood with reference to its use in an operative procedure as shown in FIG. 2. Following the determination of a proposed lesion site within a heart muscle 28 for example, the catheter 14 is directed through a blood vessel 30 to a region within the heart, such as an auricle, where the lesion will be made. The thermally-transmissive region 26 is placed proximate to the tissue to be ablated. The thermally-transmissive region of the catheter may be deformed to conform to the curvature of the tissue before, during, or after placement against the tissue. The controller 16 allows or causes cryogenic fluid to flow from the cryogenic fluid supply 10 to the fluid path in the catheter 14 and thence to the thermally-transmissive region 26 to ablate the desired area or to cold map along the same tissue area. In one embodiment (e.g., FIG. 12) a first conduit is concentric within a second conduit and cooling fluid travels to a thermally-transmissive region proximate a closed distal end of the catheter through a first conduit (fluid path) and is exhausted from the catheter through the second conduit (fluid path).

Having described the function of the cryogenic catheter 14 and its use in a system context, several exemplary embodiments of the thermally-transmissive region 26 of the catheter are now described in greater detail. FIGS. 3, 4, 5, 12-16 and 18 illustrate embodiments of the catheter, or portions thereof, having two or more thermally-transmissive segments in a spaced-apart relationship. Each of the illustrated catheters includes a closed tip 32 that can include a thermally-transmissive material.

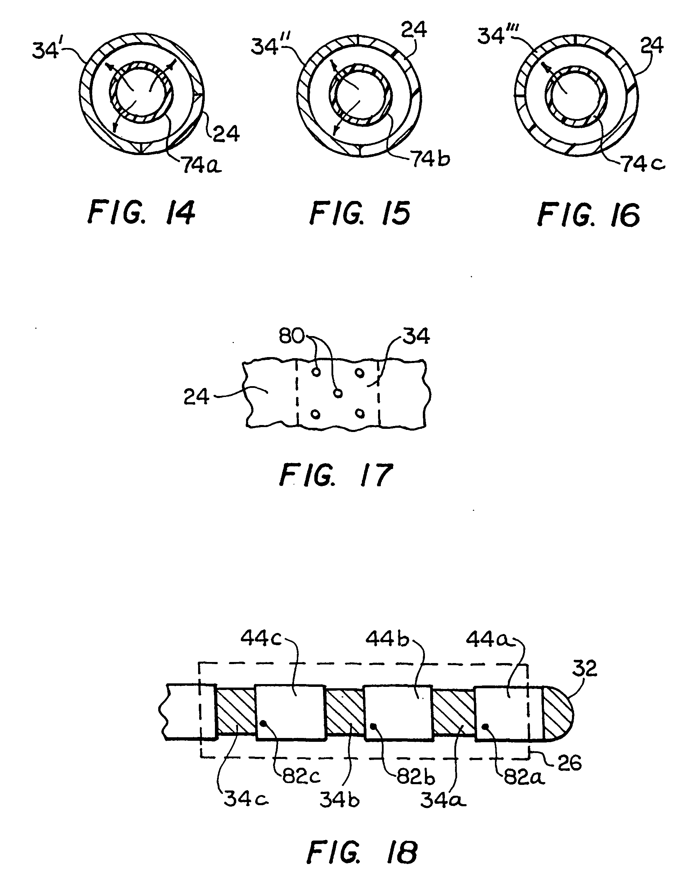

Referring specifically to the embodiment depicted in FIG. 3, multiple thermally-transmissive elements 34 are integral with a distal portion of a catheter. Each of the thermally-transmissive elements 34 includes a first side or face 36 (shown in FIGS. 12 and 13) exposed to a cryogenic fluid path and cryogenic fluid (shown by arrows) and a second side or face 38 exposed to points exterior to the catheter. As shown in FIG. 13, the first side 36 and/or second side 38 of any or all of the thermally-transmissive elements 34 can be substantially flush with, recessed below, or protruding from the inner surface 40 and outer surface 42 of a portion of the catheter. The thermally-transmissive elements 34 are separated by flexible portions of material 44 than can range from slightly less thermally-transmissive than the adjacent thermally-transmissive elements to substantially less thermally-transmissive than the adjacent elements. In the illustrated embodiment of FIG. 3, the thermally-transmissive elements 34 are annular, cylindrical elements which are made of gold-plated copper or bronze. Thermocouples 35 can be associated with one or more of the elements 34 and the tip 32. The thermally-transmissive elements 34 can be completely exposed, embedded, or a combination thereof along the full 360° of the catheter's circumference. In certain applications the thermally-transmissive elements traverse or define less than 360° of the catheter's circumference as shown in FIGS. 14-16 and as described below. The longitudinal width of each thermally-transmissive element 34, the spacing between elements, the material thickness, and the material composition are matched with a selected cooling fluid and fluid delivery pressure to produce overlapping cold regions which produce a linear lesion.

The embodiment illustrated in FIG. 4 is substantially identical to the embodiment of FIG. 3, however, at least one of the thermally-transmissive elements 34 includes a first open end 46 that defines a first plane and a second open end 48 that defines a second plane, wherein the first and second planes intersect to give the annular elements a wedge-like appearance. Such a configuration permits adjacent thermally-transmissive elements 34 to be positioned very closely together, but it can limit the possibilities for deforming the thermally-transmissive region 26, which, in this embodiment, is flexible in the direction indicated by the arrow.

With respect to the embodiments shown in both FIGS. 3 and 4, the thermally-transmissive elements 34 are substantially rigid and are separated and/or joined by a flexible material 44. However, in other embodiments the thermally-transmissive elements 34 are flexible and are interdigitated with either rigid or flexible segments. FIG. 5, for example, illustrates an embodiment of the cryogenic catheter having three thermally-transmissive elements 34 that are flexible. The flexibility is provided by a folded or bellows-like structure 50. In addition to being shapable, a metal bellows can have enough stiffness to retain a selected shape after a deforming or bending step.

Instead of, or in addition to, flexible, thermally-transmissive elements 34 and/or flexible material 44 between elements, the distal tip 32 (or a portion thereof) can be deformable. For example, FIG. 6 illustrates a tip 32 having thermally-transmissive, flexible, bellows 50.

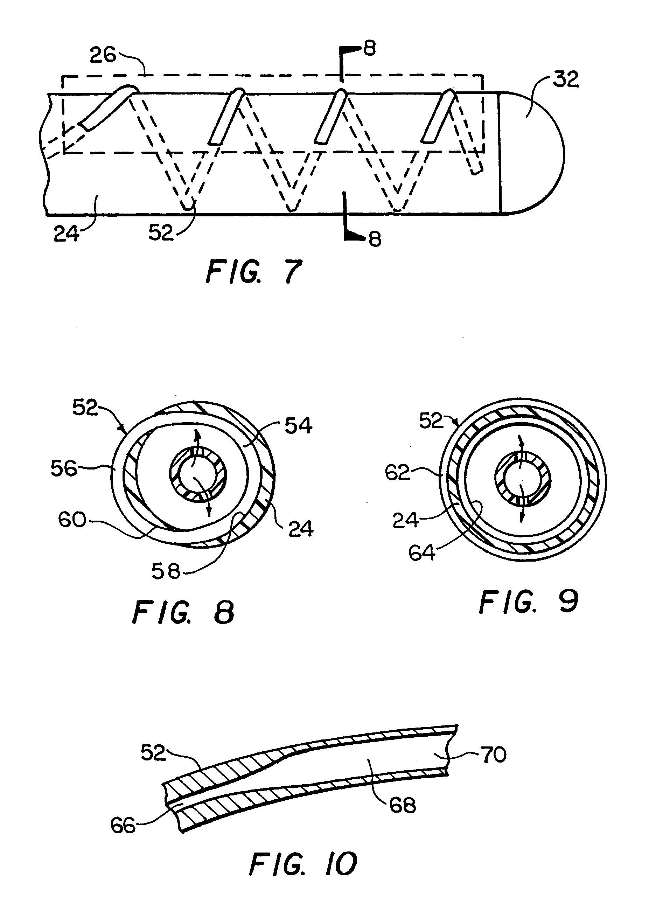

Referring now to FIGS. 7-10, a different approach is shown for providing multiple thermally-transmissive segments in a spaced-apart relationship. FIG. 7 illustrates a catheter embodiment having an elongate, thermally-transmissive region 26 that includes a helical coil 52 at least partially embedded in the flexible member 24. As shown in FIG. 8, at least a first portion 54 of the helical coil 52 is exposed to a fluid path within the flexible member 24 and a second portion 56 of the helical coil is exposed to the exterior of the flexible member. As described above with respect to FIG. 13, the first portion 54 of the coil can be substantially flush with, recessed below, or protruding from an inner surface 58 of the flexible member 24. Similarly, the second portion 56 of the coil 52 can be substantially flush with, recessed below, or protruding from an outer surface 60 of the flexible member 24.

In the embodiment of FIG. 8, the second portion 56 of the coil 52 is exposed along only a portion of the outer circumference of the flexible member 24 to define a longitudinally-elongate, thermally-transmissive region 26. This configuration can be provided by eccentrically mating the helical coil 52 to the catheter so that the longitudinal axis of the coil and the longitudinal axis of the catheter are substantially parallel. The eccentric positioning of the coil 52 provides excellent cooling performance because the surface area available for thermal exchange between the first portion 54 of coil and the cryogenic fluid is greater than the surface area available for thermal exchange between the second portion 56 of the coil and adjacent tissue where cooling power is delivered by each exposed coil portion to provide a linear lesion.

Referring now to FIG. 9, an alternative embodiment is shown wherein a first portion 62 of the coil 52 is exposed around the entire circumference of the flexible member 24, and a second portion 64 is exposed to a fluid path around the inner surface of the flexible member 24. This is achieved by having the longitudinal axis of the helical coil 52 co-axial with the longitudinal axis of the catheter.

In the embodiments illustrated in FIGS. 7-9, the coil 52 is solid. However, in other embodiments the coil can be an elongate, hollow, gas expansion chamber. For example, FIG. 10 illustrates a portion of a helical coil 52 that includes a passage that defines at least a portion of a fluid path through a flexible member of the catheter. The coil 52 defines a first fluid path diameter at a fluid entry point 66 and a second fluid path diameter that is greater than the first fluid path diameter at a gas expansion or boiling location 68. Gas escaping from a fluid exit point 70 can be exhausted through an open central region of the coil and/or another passage through the flexible member 24.

FIG. 11 illustrates an embodiment of the catheter wherein a continuous, elongate, thermally-transmissive strip 72 is longitudinally integrated with a flexible member 24. The strip can include a bellows-like structure. As described above with respect to other embodiments, a first portion of the strip can be substantially flush with, recessed below, or protrude from the outer surface of the flexible member. Similarly, a second portion of the strip can be substantially flush with, recessed below, or protrude from an inner surface of the flexible member.

Referring now to FIG. 12, an embodiment of the catheter is illustrated having a second or inner flexible member 74 concentric within a first or outer flexible member 24, wherein the second flexible member defines a fluid path to the thermally-transmissive region 26. The inner member 74 can include a single opening 76 at or near the tip 32. Cryogenic fluid is expelled from the opening 76 and returns to the proximal end of the catheter along a fluid path defined by the outer wall of the inner member 74 and the inner wall of the outer member 24. This fluid path configuration is also partially illustrated in FIGS. 8, 9, and 13. Alternatively, as also shown in FIG. 12, the inner member 74 can be provided with multiple openings 78 proximate to and/or aligned with the inner face of one or more thermally-transmissive elements 34 to achieve more uniform cooling across the entire elongate, thermally-transmissive region 26.

Referring now to FIGS. 14-16, sectional views of catheter embodiments are illustrated to show alternative configurations for thermally-transmissive elements. The previously described thermally-transmissive elements 34 are arcuate and form complete and continuous 360 degree structures that traverse the complete circumference of the catheter, notwithstanding being flush with, depressed below, or raised above the outermost surface of the flexible member 24. However, the arcuate elements 34', 34", and 34"' illustrated in FIGS. 14-16, respectively, traverse less than 360 degrees of the circumference of the first flexible member and do not form complete loops. For example, in FIG. 14, element 34' defines an approximately 270 degree arc. In FIG. 15 the thermally-transmissive element 34" defines an approximately 180 degree arc; and in FIG. 16, the thermally-transmissive element 34'" defines an approximately 90 degree arc. A catheter can include combinations of element types, such a complete ring or loop element, a 270 degree element and a 180 degree element as desired to define a thermally transmissive region. In addition to the having applicability with respect to rigid thermally-transmissive elements, the bellows-like elements can also be less than 360 degrees.

The less than 360 degree arcuate elements provide unique functional benefits with respect to thermal transfer and flexibility of the thermally-transmissive region. For example, because the portion of the catheter between the opposing ends of element 34', 34", 34"' does not include a rigid structure, but rather only the resilient material of flexible member 24, the thermally-transmissive region of the catheter can be more tightly curved (gap between ends inward and element facing outward) than it could with complete 360 degree structures, especially if the elements are relatively long longitudinally.

The inner member 74 can be adapted to direct cooling fluid at only the thermally transmissive element(s) and the shape and/or the number of openings for cooling fluid can be configured differently depending on the length of the arc defined by the thermally-transmissive element(s). For example, FIG. 14 illustrates an embodiment of the inner member having three openings opposing the thermally transmissive element 34'; FIG. 15 illustrates two openings for a smaller arc; and FIG. 16 discloses a single opening for an even smaller arc.

Another advantage to providing one or more thermally-transmissive elements that have a less than 360 degree configuration is that limiting the span of the elements to a desired lesion width, or somewhat greater than a desired lesion width, reduces the thermal load on the system and/or permits colder temperatures to be achieved than with respect to a complete 360 degree structure. Unnecessary and perhaps undesirable cooling does not occur at any other location along the catheter except at an elongate region of predetermined width. A similar effect can also be achieved by providing a non-circular 360 degree element or by eccentrically mounting a circular 360 degree element with respect to the flexible member, wherein a portion of the 360 degree element is embedded within the wall of the flexible member or otherwise insulated from the cryogenic fluid path in a manner similar to that shown in FIG. 8.

Referring now to FIG. 17, a portion of the inner face of an outer flexible member showing thermal transfer pins 80 protruding from the inner face of a thermally-transmissive element 34. The pins permit thermal transfer through the flexible member 24. As with the other features of the invention, the pins are equally suitable for complete 360 degree element structures or less than 360 degree structures.

Referring now to FIG. 18, yet another embodiment of the catheter is shown wherein rigid metal rings 34a-c are interdigitated with flexible, segments 44a-c to define a first flexible member and a thermally-transmissive region approximately one inch in length. A second flexible member is concentric within the first flexible member and has an outlet for cryogenic fluid at its distal end. Thermocouples 82a-c can be associated with one or more of the rings 34a-c.

It has been described above how the thermal loading of a cooling system can be reduced by providing thermally-transmissive elements that span less than 360 degrees. However, the thermal loading can also be reduced by sequentially cooling the tip and rings that comprise the thermally-transmissive region. One way to sequentially cool the tip and rings is to modulate the pressure of the cooling fluid along the fluid path through the flexible member, This modulation can be performed by the fluid controller which can be programmed to increase and decrease the pressure of the fluid by predetermined pressure increments over predetermined time intervals. When the cryogenic fluid is a liquid that provides cooling by changing phase from liquid to gas, the change of pressure alters the physical location along the fluid path where the phase change takes place and concomitantly changes the point of coldest temperature along the thermally-transmissive region. Thus, varying the pressure of the fluid can provide a moving ice-formation "front" along the catheter, enabling the creation of a linear lesion.

Therefore, a method (not claimed) of forming an elongate tissue lesion can include the following steps using any of the above described catheters having an elongate, thermally-transmissive region. In a first step a cryogenic fluid is introduced into the flexible member at a first predetermined pressure. Next, the pressure of the cryogenic fluid is incrementally increased within the flexible member until a second predetermined pressure is achieved. Similarly, the pressure of the cryogenic fluid within the flexible member can be decreased incrementally from the second predetermined pressure to the first predetermined pressure, wherein the steps of incrementally increasing and decreasing the pressure define a thermal cycle. Typically, from one to eight thermal cycles are required to achieve a desired therapeutic effect. In an exemplary method, about ten increments of about five seconds in duration are selected and pressure is increased by about 20 to 40 pounds per square inch in each increment. Thus, using this method an elongate lesion can be created in less than 20 minutes.

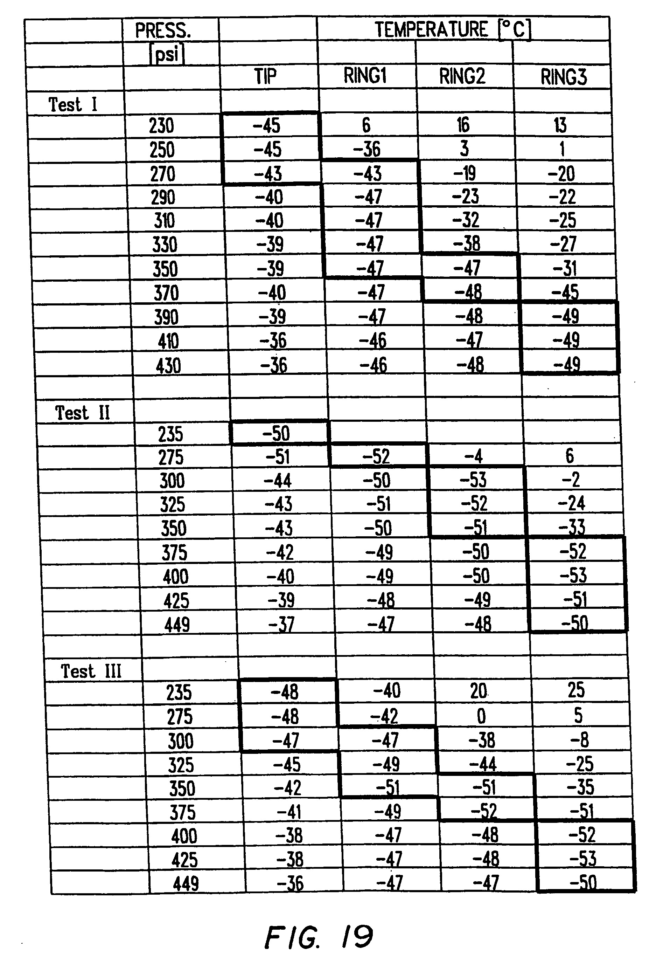

FIG. 19 is a table that illustrates sequential cooling in a catheter as described above having a thermally-transmissive region that includes a tip and three elements or rings. The table illustrates three tests conducted in a still bath at 37°C, using AZ-20 as the cryogenic fluid. Associated with each pressure increment are measured temperatures at the tip, first ring, second ring, and third ring. The shaded region illustrates the sequential movement of a target temperature range (upper -40's to low -50's) in response to a change in pressure. Although values are only provided for three rings, a similar effect and pattern is obtained with more than three rings or elements.

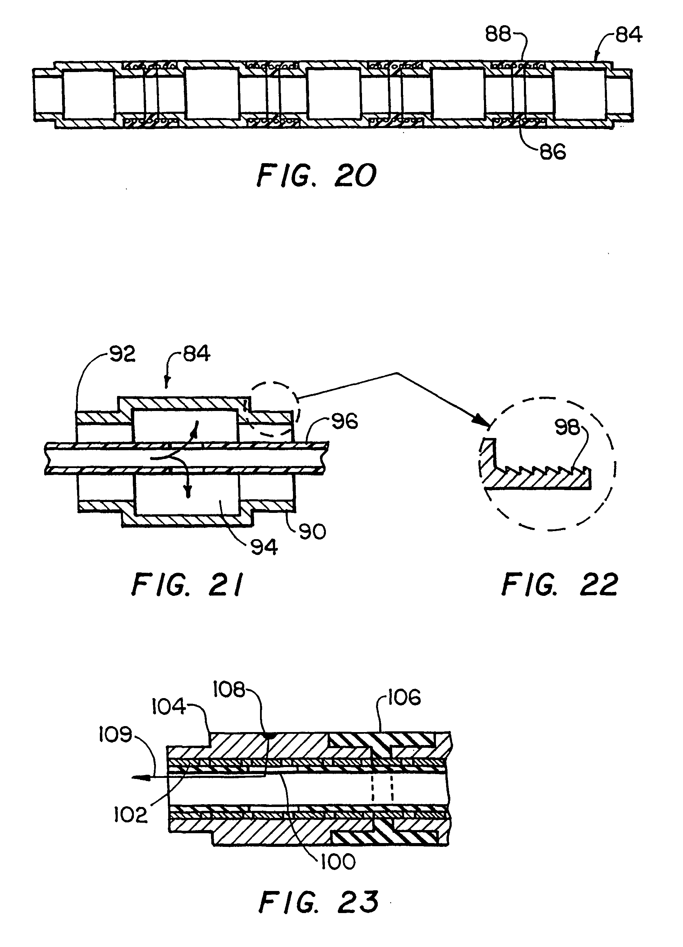

Turning now to FIG. 20, a thermally-transmissive portion of another embodiment of a medical device or structure such as a catheter is illustrated in a sectional view. The structure can include an inner passage or lumen as described above with respect to other embodiments, but which is not shown in this illustration for purposes of clarity. Thus, the illustrated portion is the outer passage or lumen that defines an elongate ablation region. Thermally-transmissive elements 84, such as gold plated copper, are joined to adjacent elements by resilient connecting elements 86, such as a stainless steel springs welded to the ends of the elements 84. A resilient bio-compatible material 88 covers the connecting elements 86 and the interstices between adjacent thermally-transmissive elements. In an exemplary embodiment, the material 88 is vulcanized silicone. It should be noted in the illustration that the surface of the elements 84 is contiguous and co-planar with the material 88 to provide a smooth outer surface.

FIG. 21 illustrates a single thermally-transmissive element 84 having reduced diameter ends 90 and 92. The wider central portion 94 provides an expansion chamber for gas (shown by arrows) exiting an apertured inner passage 96. FIG. 22 shows additional detail of the end 90 of the element 84. The end 90 is textured, such as by providing serrations 98, to provide a good adhesion surface for the material 88.

Referring now to FIG. 23, a thermally-transmissive portion of yet another embodiment of a flexible cryogenic structure is illustrated in a sectional view. In this embodiment an inner, apertured structure 100 has a flat wire 102 wrapped around it in a spiral manner. Thermally-transmissive segments 104 are disposed upon the wire 102 in a spaced-apart relationship, and a flexible, bio-compatible material 106 fills the interstices between segments 104. A thermocouple 108 can be associated with each segment 104, A wire 109 connects the thermocouple 108 to instrumentation near the proximal end of the structure, The exterior surface of the structure is smooth, and the structure can include 3 to 12 segments 104. In an exemplary embodiment the inner structure 100 is made of PTFE, the material 106 is 33 D Pebax, and the wire 102 is stainless steel or Nitinol. An apertured inner passage (similar to that shown in FIG. 21) is placed within the structure.

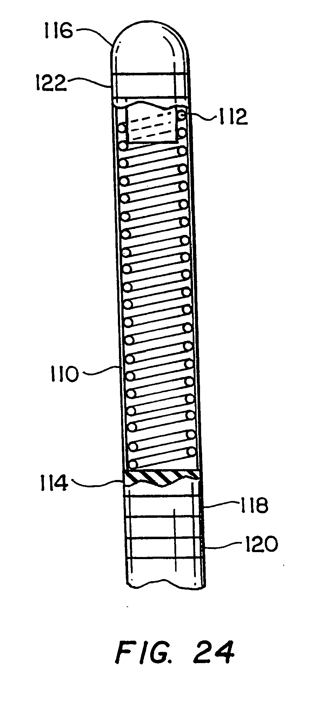

FIG. 24 illustrates still another embodiment of a cryogenic cooling structure that includes a surface or wall 110 including a polymer or elastomer that is thin enough to permit thermal transfer. For example, polyamide, PET, or PTFE having a thickness of a typical angioplasty balloon or less (below 0.006 inches) provides acceptable thermal transfer. However, the thinness of the wall 110 allows it to readily collapse or otherwise deform under vacuum or near vacuum conditions applied to evacuate fluid/gas from the structure. Accordingly, the structure is provided with one or more supporting elements 112 such as a spring. The cooling structure is illustrated in association with a catheter 114 having a closed distal tip 116 and mono or bipolar ECG rings 118, 120, 122. The thermally-transmissive region is approximately 30 mm in length and is effective for thermal transfer over its entire circumference. However, as illustrated in FIG. 11, the thermally-transmissive region can be confined to specific region(s) of the device's circumference.

A variety of modifications and variations of the present invention within the scope of the appended claims are possible in light of the above teachings.

Furthermore, although some of the illustrated devices are particularly well suited for cardiac procedures, the same embodiments and others are equally suited to other organs and/or any body portion that would benefit from the application of thermal energy. It is understood that, within the scope of the appended claims, the present invention may be practiced otherwise than as specifically described hereinabove.

What is claimed is:

## Claims

1. A cryogenic catheter (14) including a catheter body that is both elongated and flexible to be inserted into the body of a patient and has a thermally transmissive region (26) adapted to treat tissue by heat conductive cooling contact, wherein a cryogenic fluid path extends through the thermally transmissive region (26) of the catheter body to cryogenically cool the thermally transmissive region (26),
said catheter being **characterized in that** the thermally transmissive region (26) includes a helical coil (52) with a thermally transmissive material deposited on the helical coil (52) and is capable of effectively contacting and cryogenically cooling a linear lesion of tissue.

2. The cryogenic catheter of claim 1, wherein the thermally-transmissive region (26) is deformable.

3. The cryogenic catheter of claim 1, wherein the helical coil (52) is at least partially embedded in the catheter body, preferably wherein at least a first portion of the helical coil (52) is exposed to the cryogenic fluid path and a second portion of the helical coil (52) is exposed to points exterior to the catheter body, and preferably wherein the helical coil (52) includes a passage that defines at least a portion of the cryogenic fluid path, and may include a cryogenic fluid entry point leading to an expansion location within the helical coil (52).

4. The cryogenic catheter of claim 1, wherein the elongate, thermally-transmissive region (26) includes a thermally-transmissive flexible material, the thermally-transmissive material comprising: preferably a material selected from the group consisting of polyamide, PET and PTFE, and having preferably a thickness less than 0.006 inches.

5. The cryogenic catheter of claim 4, wherein the thermally-transmissive region (26) includes a plurality of thermally-transmissive elements (34) disposed on the helical coil (52), the helical coil including a flattened metal.

6. The cryogenic catheter of claim 1, further comprising an ECG ring proximate the thermally-transmissive region (26).

7. The cryogenic catheter of claim 1, further comprising a cryogenic fluid supply (10) in communication with the cryogenic fluid path; and
a fluid controller (16) interposed between the cryogenic fluid path and the cryogenic fluid supply (10), for regulating the flow of the cryogenic fluid into the cryogenic fluid path.

8. The cryogenic catheter of claim 7 wherein the fluid is introducible into the cryogenic fluid path under pressure and wherein the fluid controller (16) is programmable to increase and decrease the pressure of the fluid by predetermined pressure increments over predetermined time intervals.

## Patentansprüche

1. Ein kryogener Katheter (14), der einen Katheterkörper einschließt, welcher sowohl langgestreckt als auch biegsam ist, um in den Körper eines Patienten eingeführt zu werden, und einen wärmedurchlässigen Bereich (26) hat, ausgebildet, um Gewebe durch wärmeleitenden Kühlungskontakt zu behandeln, wobei sich ein kryogener Fluidpfad durch den warmedurchlässigen Bereich (26) des Katheterkörpers erstreckt, um den wärmedurchlässigen Bereich (26) kryogen zu kühlen,
wobei der Katheter **dadurch gekennzeichnet ist, dass** der wärmedurchlässige Bereich (26) eine Spiralwindung (52) mit einem wärmedurchlässigen Material einschließt, das auf die Spiralwindung (52) aufgetragen wird, und in der Lage ist, eine lineare Läsion von Gewebe wirksam zu berühren und kryogen zu kühlen.

2. Der kryogene Katheter gemäß Anspruch 1, worin der wärmedurchlässige Bereich (26) verformbar ist.

3. Der kryogene Katheter gemäß Anspruch 1, worin die Spiralwindung (52) zumindest teilweise in den Katheterkörper eingebettet ist, worin vorzugsweise zumindest ein erster Abschnitt der Spiralwindung (52) dem kryogenen Fluidpfad ausgesetzt ist und ein zweiter Abschnitt der Spiralwindung (52) Punkten außet-halb des Katheterkörpers ausgesetzt ist und worin die Spiralwindung (52) vorzugsweise einen Durchgang einschließt, der zumindest einen Abschnitt des kryogenen Fluidpfads bestimmt, und einen kryogenen Fluideintrittspunkt einschließen kann, der zu einem Expansionsort innerhalb der Spiralwindung (52) führt.

4. Der kryogene Katheter gemäß Anspruch 1, worin der lang gestreckte, wärmedurchlässige Bereich (26) ein wärmedurchlässiges biegsames Material einschließt und das wärmedurchlässige Material vorzugsweise ein Material umfasst, das gewählt ist aus der Gruppe bestehend aus Polyamid, PET und PTFE, und vorzugsweise eine Dicke von weniger als 0,006 Zoll hat.

5. Der kryogene Katheter gemäß Anspruch 4, worin der wärmedurchlässige Bereich (26) eine Vielzahl wärmedurchlässiger Elemente (34) einschließt, die auf der Spiralwindung (52) angeordnet sind, wobei die Spiralwindung ein abgeflachtes Metall einschließt,

6. Der kryogene Katheter gemäß Anspruch 1, der weiter einen ECG-Ring nahe dem wärmedurchlässigen Bereich (26) umfasst.

7. Der kryogene Katheter gemäß Anspruch 1, der weiter eine kryogene Fluidzufuhr (10) in Kommunikation mit dem kryogenen Fluidpfad umfasst; und
eine Fluidsteuerung (16), angeordnet zwischen dem kryogenen Fluidpfad und der kryogenen Fluidzufuhr (10), zur Regulierung des Flusses des kryogenen Fluids in den kryogenen Fluidpfad.

8. Der kryogene Katheter gemäß Anspruch 7, worin das Fluid unter Druck in den kryogenen Fluidpfad eingeführt werden kann und worin die Fluidsteuerung (16) programmiert werden kann, um den Druck des Fluids durch vordefinierte Druckstufen über vordefinierte Zeitintervalle zu erhöhen und zu senken.

## Revendications

1. Cathéter cryogénique (14), incluant un corps de cathéter, à la fois allongé et flexible pour être inséré dans le corps d'un patient et présentant une région thermiquement transmissive (26), adaptée pour traiter des tissus par un contact de refroidissement thermo-conducteur, dans lequel un chemin à fluide cryogénique s'étend à travers la région thermiquement transmissive (26) du corps de cathéter, pour refroidir cryogéniquement la région thermiquement transmissive (26),
ledit cathéter étant **caractérisé en ce que** la région thermiquement transmissive (26) comprend un serpentin hélicoïdal (52) avec un matériau thermiquement transmissif, déposé sur le serpentin hélicoïdal (52) et est capable d'entrer efficacement en contact et de refroidir cryogéniquement une lésion linéaire du tissu.

2. Cathéter cryogénique selon la revendication 1, dans lequel la région thermiquement transmissive (26) est déformable,

3. Cathéter cryogénique selon la revendication 1, dans lequel le serpentin hélicoïdal (52) est au moins partiellement intégré dans le corps de cathéter, de préférence dans lequel au moins une première partie du serpentin hélicoïdal (52) est exposée à des points extérieurs au corps de cathéter, et de préférence le serpentin hélicoïdal (52) comprend un passage définissant au moins une partie du chemin à fluide cryogénique, et peut comprendre un point d'entrée de fluide cryogénique, menant à un emplacement d'expansion à l'intérieur du serpentin hélicoïdal (52).

4. Cathéter cryogénique selon la revendication 1, dans lequel la région thermiquement transmissive (26) allongée comprend un matériau flexible thermiquement transmissif, le matériau thermiquement transmissif comprenant de préférence un matériau sélectionné dans le groupe composé de polyamide, PET et PTFE, et ayant de préférence une épaisseur inférieure à 0,1524 mm (0,006 pouce).

5. Cathéter cryogénique selon la revendication 4, dans lequel la région thermiquement transmissive (26) comprend une pluralité d'éléments thermiquement transmissifs (34) disposés sur le serpentin hélicoïdal (52), le serpentin hélicoïdal comprenant un métal aplati.

6. Cathéter cryogénique selon la revendication 1, comprenant en outre une boucle d'ECG, à proximité de la région thermiquement transmissive (26).

7. Cathéter cryogénique selon la revendication 1, comprenant en outre une alimentation en fluide cryogénique (10), en communication avec le chemin à fluide cryogénique ; et
un contrôleur de fluide (16), interposé entre le chemin à fluide cryogénique et l'alimentation en fluide cryogénique (10), pour réguler l'écoulement de fluide cryogénique dans le chemin à fluide cryogénique.

8. Cathéter cryogénique selon la revendication 7, dans lequel le fluide est susceptible d'être introduit dans le chemin à fluide cryogénique sous pression, et dans lequel le contrôleur de fluide (16) est programmable, pour augmenter et diminuer la pression du fluide, en procédant par incréments de pression prédéterminées, sur des intervalles de temps prédéterminés.
